# EUROPEAN PATENT APPLICATION

(11) **EP 2 559 447 A1**
(43) Date of publication of application: **20.02.2013**
(21) Application number: 11177679.5
(22) Date of filing: 16.08.2011
(51) Int. Cl.: A61M 5/00, A61M 5/32, A61M 5/34

(54) **Single-use needle assembly and storage device**

(71) Applicant: Sanofi-Aventis Deutschland GmbH, 65929 Frankfurt (DE)
(72) Inventor: The designation of the inventor has not yet been filed

(57) **Abstract**

Described is a needle assembly (5) comprising a needle (10) and a hub (15, 30, 40, 55) coupled to the needle. The hub (15, 30, 40) includes an engagement member (20, 45, 60) adapted to engage an attachment arrangement on a medicament delivery device and a stop member (25, 50, 65) adapted to abut the attachment arrangement to prevent proximal movement of the needle assembly (5).

## Description

### Technical Field

The invention relates to a single-use needle assembly and storage device that is adapted for use with a medicament delivery device such as, for example, a syringe, a pen injector or an auto-injector.

### Background of the Invention

Patients suffering from diseases like diabetes have to frequently self-administer injections. Injection devices like auto-injectors or pen injectors have been developed to facilitate self-administering injections. Typically, such injection devices are fitted with a sterile, disposable needle assembly for each injection to minimize the risk of infections.

A conventional needle assembly consists of a hub for engaging the injection device, a double-tipped needle coupled to the hub, and a spring-biased needle shield for hiding the needle from view and covering an injection end of the needle after the injection. The conventional needle assembly also includes a protective cap to maintain sterility of the needle and prevent against inadvertent actuation. Conventional needle assemblies are often packaged loosely in boxes. Thus, a patient is required to carry a box of the needle assemblies (and a sharps disposal unit) when travelling. Similarly, loosely packing the needle assemblies does not optimally use packing space available.

Thus, there is a need for a single-use needle assembly for use with a medicament delivery device and a storage device for the single-use needle assembly.

### Summary of the Invention

It is an object of the present invention to provide a single-use needle assembly and storage device.

In an exemplary embodiment, a needle assembly comprises a needle and a hub coupled to the needle. The hub includes an engagement member adapted to engage an attachment arrangement on a medicament delivery device and a stop member adapted to abut the attachment arrangement to prevent proximal movement of the needle assembly. A width of the stop member may be substantially equal to or greater than a width of the engagement member. The engagement member and the stop member may comprise a bayonet coupling.

In an exemplary embodiment, the needle assembly further comprises an extraction member having a gripping surface.

In an exemplary embodiment, a storage device for the needle assembly comprises a body having a proximal opening and a piston axially movable in the body. An inner width of the body may be substantially equal to the width of the stop member. The storage device may further comprise a stop element adapted to prevent movement of the piston beyond a distal opening of the body. A proximal end of the piston may be adapted to abut the hub or may include one or more latches adapted to engage the hub. The latches may be biased away from a longitudinal axis of the body.

The storage device may further comprise a hook disposed on a distal end of the piston.

The storage device may further comprise a membrane disposed in the body and adapted to frictionally engage the at least one of the needle and the hub.

The proximal opening may be covered by a film.

In an exemplary embodiment, an array of a plurality of the storage devices for the needle assemblies can form a circle, a row, a spiral or a helix.

Further scope of applicability of the present invention will become apparent from the detailed description given hereinafter. However, it should be understood that the detailed description, while indicating exemplary embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
Figures 1a-d shows exemplary embodiments of a single-use needle assembly according to the present invention,
Figures 2a-c show exemplary embodiments of a storage device for a single-use needle assembly according to the present invention, and
Figures 3a-f show exemplary embodiments of arrays of storage devices for single-use needle assemblies according to the present invention.

Corresponding parts are marked with the same reference symbols in all figures.

### Detailed Description

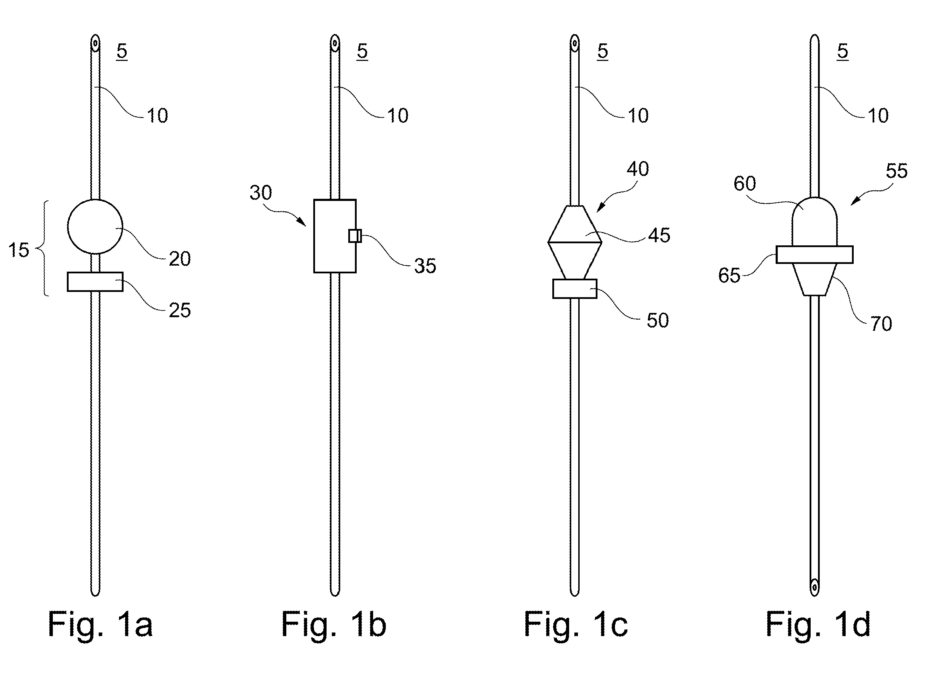

Figures 1a-d show exemplary embodiments of a single-use needle assembly 5 according to the present invention. In each exemplary embodiment, the assembly 5 includes a double-tipped needle 10 having bevelled edges at both tips. A proximal tip is adapted to pierce a septum on syringe or cartridge of medicament. A distal tip is adapted to pierce the skin (or other anatomical feature) to administer the medicament.

Each exemplary embodiment of the assembly 5 also includes a hub adapted to engage an attachment device on a medicament delivery device. In any embodiment, the hub may be overmolded on the needle 10 and/or coupled to the needle 10 on a portion of the needle 10 between the proximal and distal tips via an adhesive or welding. Further, the hub may be formed of one or more components which may be formed integrally or separately, and in the latter embodiment, separately coupled to the needle 10.

In the exemplary embodiment shown in Figure 1 a, a hub 15 includes an engagement member 20 and a stop member 25. The engagement member 20 is adapted to engage a socket in the attachment device on the medicament delivery device, and a shape and/or friction fit between the engagement member 20 and the socket prevents movement of the assembly 5 relative to the medicament delivery device. The stop member 25 prevents proximal movement of the assembly 5 during an injection procedure. The stop member 25 may also be used for removing the needle 10 after an injection, e.g., a removal device may grip the stop member 25 to allow the engagement member 20 to be separated from the socket (e.g., by pulling). In the exemplary embodiment shown in Figure 1 a, the engagement member 20 is spherical and the stop member 25 is linear, disposed perpendicular to the needle 10. A width of the stop member 25 may be equal to or greater than a diameter of the engagement member 20. The stop member 25 may be disposed distal to the engagement member 20 on the needle 10.

Figure 1 b shows another exemplary embodiment of a hub 30 which includes a bayonet coupling 35. The bayonet coupling 35 may be inserted into an attachment device on a medicament delivery device and rotated to lock the bayonet coupling 35 to the attachment device. Rotation in an opposite direction may be used to unlock the bayonet coupling 35, e.g., after an injection is complete. In this exemplary embodiment, the engagement member may be a base coupled to the needle 10 and the stop member may be a projection extending radially from the base to engage a groove in the attachment device.

Figure 1c shows another exemplary embodiment of a hub 40 which includes an engagement member 45 and a stop member 50. In this exemplary embodiment, the engagement member 45 is comprised of two, abutting conical members oriented in opposite directions, and the stop member 50 abuts the engagement member 45.

Figure 1 d shows another exemplary embodiment of a hub 55 which includes an engagement member 60, a stop member 65 and an extraction member 70. The extraction member 70 may be adapted as a gripping surface for an extraction device to remove the needle 10 from an attachment device on a medicament delivery device. For example, the extraction member 70 may include one or more grooves, slots, hooks, etc. for engaging the extraction device.

In other exemplary embodiments, the hub may be formed as a thread to mate with a corresponding thread on the attachment device of the medicament delivery device.

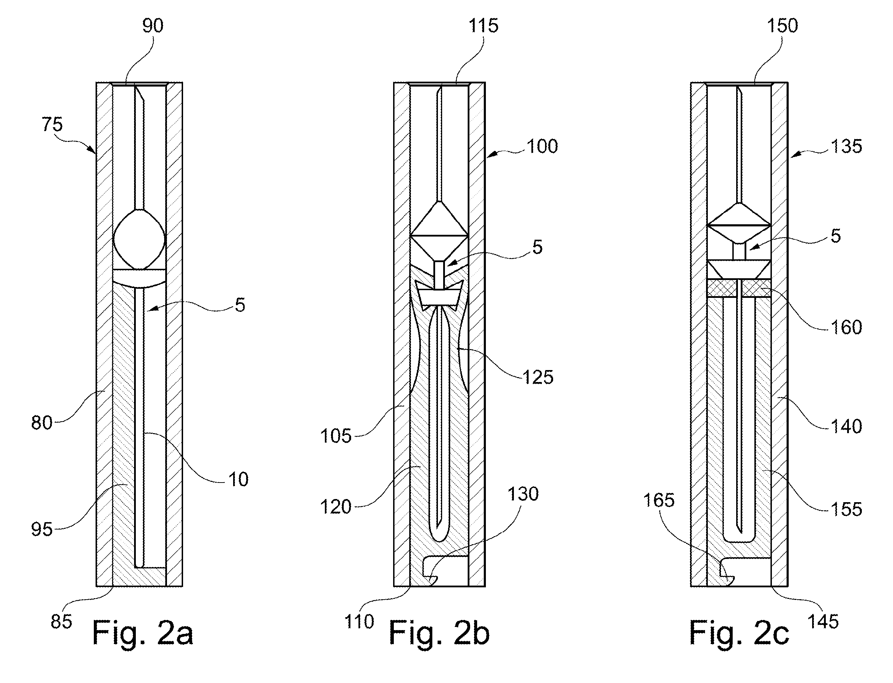

Figures 2a-c show exemplary embodiments of a storage device for a single-use needle assembly according to the present invention.

In an exemplary embodiment shown in Figure 2a, a storage device 75 for the needle assembly 5 includes a body 80, which may be cylindrical, with a distal opening 85 and a proximal opening 90. An inner width/diameter of the body 80 may be substantially equal to a width/diameter of the hub of the needle assembly 5. A piston 95 may be disposed in the body 80. In an exemplary embodiment, the piston 95 may have a L-shaped cross-section with a proximal end adapted to abut the hub of the needle assembly 5 and a distal end disposed adjacent the distal tip of the needle 10. The piston 95 may have a width/diameter substantially equal to the inner width/diameter of the body 80, such that the piston 95 fits telescopically within the body 80 and is adapted to move axially relative to the body 80. In an exemplary embodiment, the distal end of the piston 95 (or a distal end of the body 80) may include a stop element to prevent the distal end of the piston from moving past a distal end of the body 80. In an exemplary embodiment, the distal opening 85 and/or the proximal opening 90 may be sealed by a film, e.g., to maintain sterility of an unused needle assembly 5. A force may be applied to the piston 95 to push the needle assembly 5 proximally to expose the hub via the proximal opening 90, so that the assembly 5 may be engaged by the medicament delivery device. After use, the assembly 5 may be reinserted into the body 80 for storage, and the needle assembly 5 may be pushed distally into the body 80. The stop element on the distal end of the piston 95 may prevent the distal end of the needle 10 from passing through the distal opening 85 of the body 80.

In an exemplary embodiment shown in Figure 2b, a storage device 100 for the needle assembly 5 includes a body 105, which may be cylindrical, with a distal opening 110 and a proximal opening 115. An inner width/diameter of the body 105 may be substantially equal to a width/diameter of the hub of the needle assembly 5. A piston 120 may be disposed in the body 105. In an exemplary embodiment, the piston 120 may have a U-shaped cross-section with a proximal end including one or more latches 125 adapted to engage the hub of the needle assembly 5 and a distal end disposed adjacent the distal tip of the needle 10. The piston 120 may have a width/diameter substantially equal to the inner width/diameter of the body 105, such that the piston 120 fits telescopically within the body 105 and is adapted to move axially relative to the body 105. In an exemplary embodiment, the distal end of the piston 120 (or a distal end of the body 105) may include a stop element to prevent the distal end of the piston from moving past a distal end of the body 105. In an exemplary embodiment, the distal opening 110 and/or the proximal opening 115 may be sealed by a film, e.g., to maintain sterility of an unused needle assembly 5. A force may be applied to the piston 120 to push the needle assembly 5 proximally to expose the hub via the proximal opening 115, so that the assembly 5 may be engaged by the medicament delivery device. After use, the assembly 5 may be reinserted into the body 105 for storage, and the needle assembly 5 may be pushed distally into the body 105. The stop element on the distal end of the piston 120 may prevent the distal end of the needle 10 from passing through the distal opening 110 of the body 105.

In this exemplary embodiment, the one or more latches 125 may be biased away from a longitudinal axis of the body 105. When the latches 125 are in the body 105, the latches 125 may be forced inward by walls of the body 105 to engage (e.g., by shape and/or friction) the hub on the needle assembly 5 or the needle 10. When a force is applied to the piston 120 and the latches 125 are exposed via the proximal opening 115, the latches 125 splay apart, releasing the needle assembly 5 and allowing the needle assembly 5 to be engaged by the medicament delivery device.

In this exemplary embodiment, a hook 130 may be disposed at the distal end of the piston 120. After use of the needle assembly 5, the used needle assembly 5 may be deposited in the piston 120, and the piston 120 may be pulled distally so that the latches 125 may engage the hub on the needle assembly 5. Thus, the used needle assembly 5 may be secured within the body 105.

In an exemplary embodiment shown in Figure 2c, a storage device 135 for the needle assembly 5 includes a body 140, which may be cylindrical, with a distal opening 145 and a proximal opening 150. An inner width/diameter of the body 140 may be substantially equal to a width/diameter of the hub of the needle assembly 5. A piston 155 may be disposed in the body 140. In an exemplary embodiment, the piston 155 may have a U-shaped cross-section with a proximal end including a membrane 160 adapted to frictionally engage the hub of the needle assembly 5 and/or the needle 10, and a distal end disposed adjacent the distal tip of the needle 10. The piston 155 may have a width/diameter substantially equal to the inner width/diameter of the body 140, such that the piston 155 fits telescopically within the body 140 and is adapted to move axially relative to the body 140. In an exemplary embodiment, the distal end of the piston 155 (or a distal end of the body 140) may include a stop element to prevent the distal end of the piston 155 from moving past a distal end of the body 140. In an exemplary embodiment, the distal opening 145 and/or the proximal opening 150 may be sealed by a film, e.g., to maintain sterility of an unused needle assembly 5. A force may be applied to the piston 155 to push the needle assembly 5 proximally to expose the hub via the proximal opening 135, so that the assembly 5 may be engaged by the medicament delivery device and pulled from the membrane 160. After use, the assembly 5 may be reinserted into the body 140 for storage, and the needle assembly 5 may be pushed distally into the body 140. The stop element on the distal end of the piston 155 may prevent the distal end of the needle 10 from passing through the distal opening 145 of the body 105.

In this exemplary embodiment, a hook 165 may be disposed at the distal end of the piston 155. After use of the needle assembly 5, the used needle assembly 5 may be deposited in the membrane 160, and the piston 160 may be pulled distally so that the used needle assembly 5 may be secured within the body 140.

In an exemplary embodiment, the membrane 160 may be formed from silicon, foam, rubber or a polymer.

Those of skill in the art will understand that the storage device may not include a distal opening. For example, a distal end of the body may be covered, and the piston may be moved axially by pushing a lever or projection coupled to the piston which projects from a slot formed in the body.

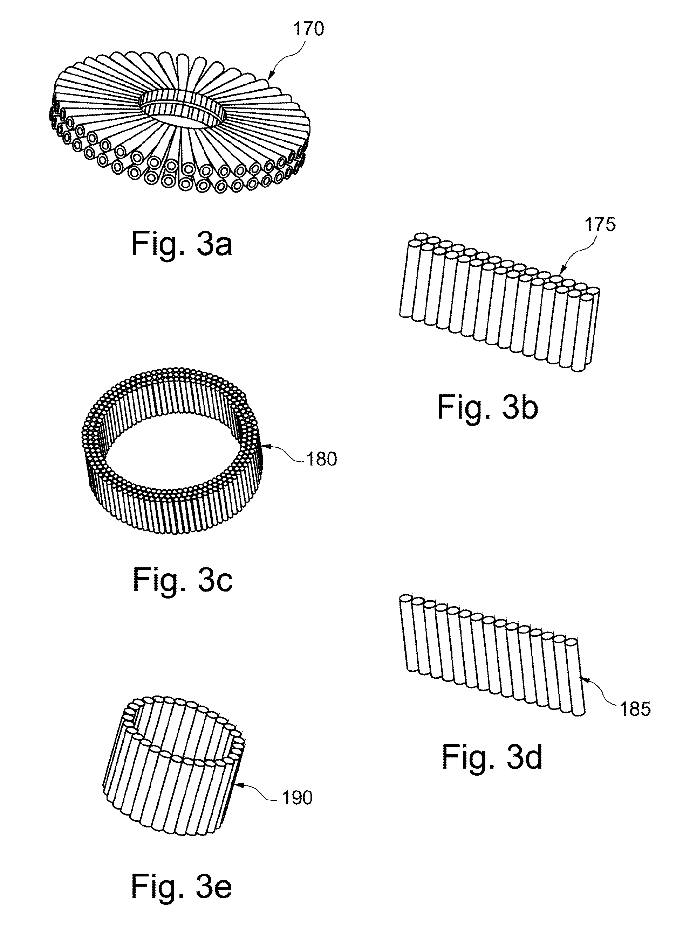

Figures 3a-f show exemplary embodiments of arrays of storage devices for single-use needle assemblies according to the present invention. Those of skill in the art will understand that the arrays may be injection molded from plastic, rubber, a polymer, etc.

In an exemplary embodiment shown in Figure 3a, an array 170 of storage devices for the needle assembly 5 may be formed as a circle of two adjacent rows, with the proximal openings of the storage devices facing away from a center of the circle. Those of skill in the art will understand that any number of rows may be utilized.

In an exemplary embodiment shown in Figure 3b, an array 175 of storage devices for the needle assembly 5 may be formed as a two adjacent rows, with the proximal openings of the storage devices facing in a first direction and the distal openings of the storage devices facing in a second direction opposite the first direction. Those of skill in the art will understand that any number of rows may be utilized.

In an exemplary embodiment shown in Figure 3c, an array 180 of storage devices for the needle assembly 5 may be formed as a belt of consecutive storage devices wound into a spiral, with the proximal openings of the storage devices facing in a first direction and the distal openings of the storage devices facing in a second direction opposite the first direction. Those of skill in the art will understand that any number of rows may be utilized.

In an exemplary embodiment shown in Figure 3d, an array 185 of storage devices for the needle assembly 5 may be formed as a row, with the proximal openings of the storage devices facing in a first direction and the distal openings of the storage devices facing in a second direction opposite the first direction. Those of skill in the art will understand that any number of rows may be utilized.

In an exemplary embodiment shown in Figure 3e, an array 190 of storage devices for the needle assembly 5 may be formed as a circle of consecutive storage devices, with the proximal openings of the storage devices facing in a first direction and the distal openings of the storage devices facing in a second direction opposite the first direction. Those of skill in the art will understand that any number of rows may be utilized.

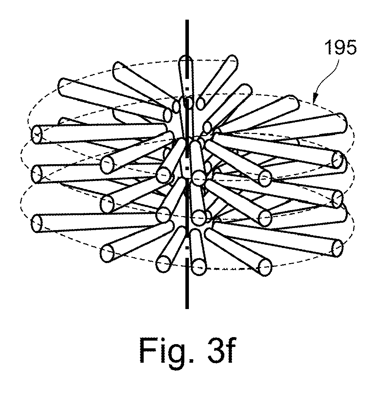

In an exemplary embodiment shown in Figure 3f, an array 195 of storage devices for the needle assembly 5 may be formed as a helix, with the proximal openings of the storage devices facing away from a center axis of the helix.

Those of skill in the art will understand the modifications (additions and/or removals) of various components of the device and/or system and embodiment described herein may be made without departing from the full scope and spirit of the present invention, which encompass such modifications and any and all equivalents thereof.

## Claims

1. A needle assembly (5) comprising:
a needle (10); and
a hub (15, 30, 40, 55) coupled to the needle, the hub (15, 30, 40) including:
an engagement member (20, 45, 60) adapted to engage an attachment arrangement on a medicament delivery device; and
a stop member (25, 50, 65) adapted to abut the attachment arrangement to prevent proximal movement of the needle assembly (5).

2. The needle assembly (5) according to claim 1, wherein a width of the stop member (25, 50, 65) is substantially equal to or greater than a width of the engagement member (20, 45, 60).

3. The needle assembly (5) according to claim 1, wherein the engagement member (20, 45, 60) and the stop member (25, 50, 65) comprise a bayonet coupling (35).

4. The needle assembly (5) according to any of the preceding claims, wherein the hub (15, 30, 40, 55) further comprises:
an extraction member (70) having a gripping surface.

5. A storage device (75, 100, 135) for the needle assembly (5) according to any of the preceding claims, the storage device (75, 100, 135) comprising:
a body (80, 105, 140) having a proximal opening (90, 115, 150); and
a piston (95, 120, 155) axially movable in the body (80, 105, 140).

6. The storage device (75, 100, 135) according to claim 5, wherein an inner width of the body (80, 105, 140) is substantially equal to the width of the stop member (25, 50, 65).

7. The storage device (75, 100, 135) according to claims 5 or 6, further comprising:
a stop element adapted to prevent movement of the piston (95, 120, 155) beyond a distal opening (85, 110, 145) of the body (80, 105, 140).

8. The storage device (75, 100, 135) according to any of claims 5, 6 or 7, wherein a proximal end of the piston (95, 120, 155) is adapted to abut the hub (15, 30, 40, 55).

9. The storage device (75, 100, 135) according to any of claims 5, 6 or 7, wherein a proximal end of the piston (95, 120, 155) includes one or more latches (125) adapted to engage the hub (15, 30, 40, 55).

10. The storage device (75, 100, 135) according to claim 9, wherein the one or more latches (125) are biased away from a longitudinal axis of the body (80, 105, 140).

11. The storage device (75, 100, 135) according to any of claims 5 to 10, further comprising:
a hook (130, 165) disposed on a distal end of the piston (95, 120, 155).

12. The storage device (75, 100, 135) according to any of claims 5 to 10, further comprising:
a membrane (160) disposed in the body (80, 105, 140) and adapted to frictionally engage the at least one of the needle (10) and the hub (15, 30, 40, 55).

13. The storage device (75, 100, 135) according to any of claims 5 to 10, wherein the proximal opening (90, 115, 150) is covered by a film.

14. An array (170, 175, 180, 185, 190) of a plurality of the storage devices according to any of claims 5-12, the array (170, 175, 180, 185, 190) comprising a circle, a row, a spiral or a helix.
